(19) Europäisches Patentamt
European Patent Office
Office européen des brevets

(11) Publication number: **0 101 517 B1**

# (12) EUROPEAN PATENT SPECIFICATION

(45) Date of publication of patent specification: **29.10.86**

(51) Int. Cl.$^4$: **C 07 D 487/04**, C 07 D 487/14

(21) Application number: **82900567.7**

(22) Date of filing: **19.02.82**

(86) International application number: **PCT/JP82/00045**

(87) International publication number: **WO 83/02944 01.09.83 Gazette 83/20**

## (54) NOVEL s-TRIAZOLE(1,5-a)PYRIMIDINE DERIVATIVES.

(43) Date of publication of application: **29.02.84 Bulletin 84/09**

(45) Publication of the grant of the patent: **29.10.86 Bulletin 86/44**

(84) Designated Contracting States: **FR**

(56) References cited:
**JP-A-55 051 089**
**JP-A-56 049 385**
**JP-A-57 035 592**
**JP-A-57 035 593**

**Pharmazie (1971-26-9) P. 537**

(73) Proprietor: **MOCHIDA PHARMACEUTICAL CO., LTD.**
**7, Yotsuya 1-chome**
**Shinjuku-ku Tokyo 160 (JP)**

(72) Inventor: **OHNISHI, Haruo**
**4-14, Higashifunabashi 6-chome Funabashi-shi**
**Chiba 273 (JP)**
Inventor: **KOSUZUME, Hiroshi**
**11-5, Fuyodai 3-chome Mishima-Shi**
**Shizuoka 411 (JP)**
Inventor: **SUZUKI, Yasuo**
**234-29, Ohaza-Sashima Kawaguchi-shi**
**Saitama 333 (JP)**
Inventor: **MOCHIDA, Ei**
**5-4, Komagome 2-chome Toshima-ku**
**Tokyo 170 (JP)**

(74) Representative: **Lavoix, Jean et al**
**c/o Cabinet Lavoix 2, Place D'Estienne D'Orves**
**F-75441 Paris Cedex 09 (FR)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 101 517 B1

**Description**

Technical Field

The present invention relates to s-triazolo[1,5-a]pyrimidine derivatives possessing vasodilating action, hypotensive action, inhibitory action on platelete aggregation, and lowering action on cholesterol levels in blood.

Background Art

Cardiovascular diseases, for example ischemic heart diseases, cerebrovascular diseases, atherosclerosis and hypertension, together with malignant tumors, are quite important diseases as major causes of deaths. Among these cardiovascular diseases, ischemic heart diseases and insufficiency of cerebral or peripheral circulation are generally understood to be caused by lack of nutrition and oxygen supply, which are originally caused by insufficient blood flow resulting from arterial thrombus formation or from atherosclerosis. Therefore, a drug which improves blood flow of the affected organ based on vasodilating action on ischemic area, such as coronary arteries, is effective in the treatment of these diseases. Also, a drug that inhibits platelet aggregation, which is a cause of arterial thrombus formation, or a drug that reduces elevated serum cholesterol, which causes atherosclerosis, is understood to be effective in preventing pathogenesis of these diseases.

Vasodilators, for example hydralazine, show hypotensive activity and have been frequently used in the treatment of hypertensive disorders. Recently, vasodilators have also been used in the treatment of cardiac insufficiency, based on their action to reduce peripheral vascular resistance, as reported by Itoh et al. (Nippon Rinsho, *36*(11) 51(1978)) and by Matsui et al. (Abstract No. 165, The 43rd annual congress of the Japanese Circulation Society).

JP—A—56—49385 of the Applicant and E. TENOR and R. LUDWIG, Die Pharmazie 1971, 26—9, 534—39, show s-triazolo(1,5-a)pyrimidine derivatives useful in cardiovascular diseases.

The inventors observed that cardiovascular diseases as mentioned above are related to each other and that the optimal treatment of these diseases would be such as to improve cardiovascular functions, disorders and diseases comprehensively. As a result of a search for compounds that are suitable for this purpose, the inventors have accomplished the invention based on the finding that novel s-triazolo[1,5-a]pyrimidine derivatives of the following general formula (I) possess vasodilating action, hypotensive action, inhibitory action on platelet aggregation, and reducing action on cholesterol levels in blood.

The compounds of the invention are represented by the formula:

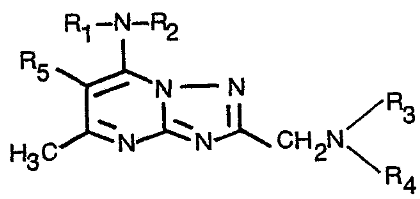

(I)

wherein

$R_1$, $R_2$, which are identical or different, each represents a straight- or branched-chained alkyl radical having from one to six carbon atoms or a hydrogen atom;

$R_3$ represents a straight- or branched-chained alkyl radical having from one to six carbon atoms;

$R_4$ represents a phenyl radical; and

$R_5$ represents a hydrogen atom or may form a $—CH_2—CH_2—$ bond together with $R_1$.

The compounds according to this invention can be prepared as follows:

For example, 3-amino-5-hydroxymethyl-s-triazole is condensed with an α-acylcarboxylic acid ester or the like under heating to obtain a 2-hydroxymethyl-7-hydroxy-s-triazolo[1,5-a]pyrimidine derivative. The resultant derivative is then halogenated with an appropriate halogenating agent to obtain a 2-halomethyl-7-halo-s-triazolo[1,5-a]pyrimidine derivative. Then the resultant halogenated derivative is reacted with an appropriate amine to obtain a s-triazolo[1,5-a]pyrimidine derivative of the present invention.

An example of such a preparation is shown overleaf

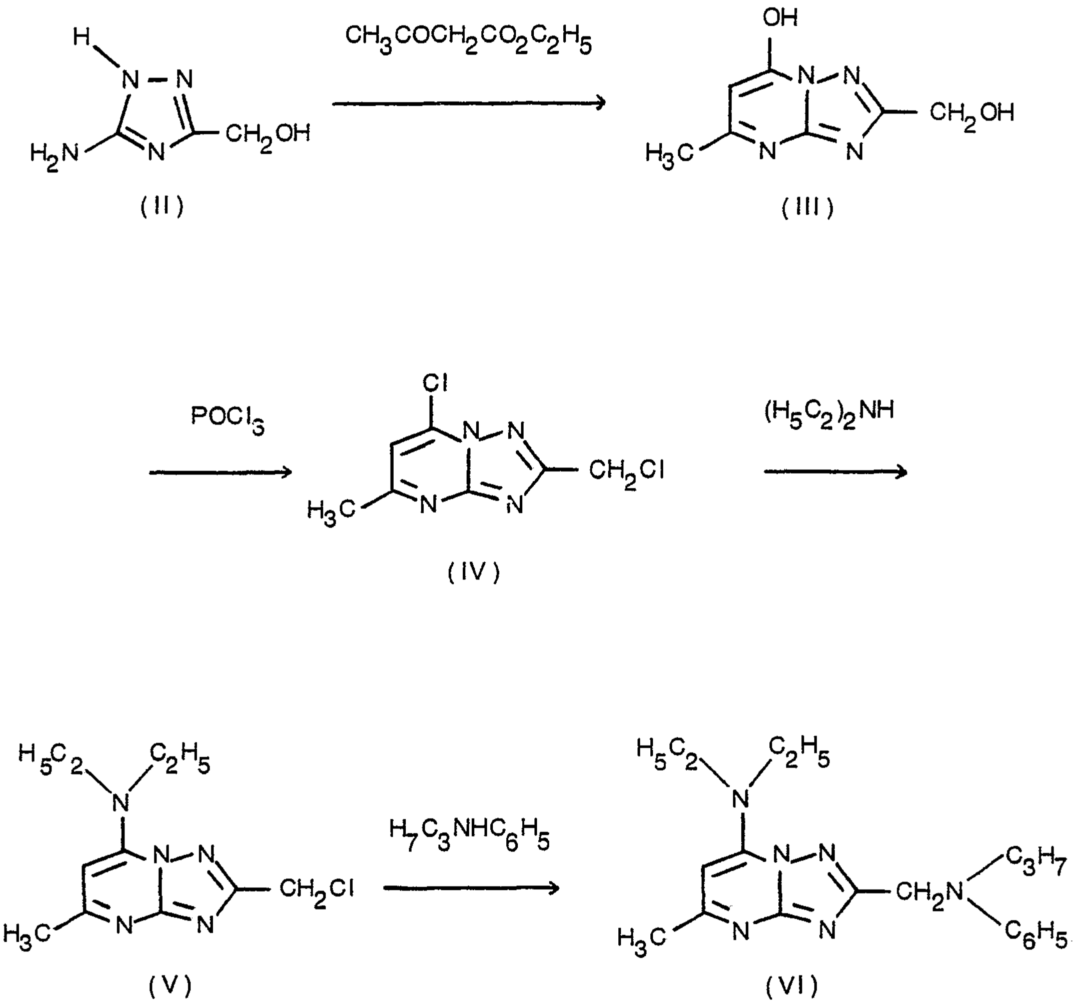


(II) (III) (IV) (V) (VI)

3-Amino-5-hydroxymethyl-s-triazole (II) together with ethyl acetoacetate is heated in the absence of solvent under neutral conditions, or refluxed under heating using a lower saturated fatty acid such as acetic acid as a solvent, to obtain 7-hydroxy-2-hydroxymethyl-5-methyl-s-triazolo[1,5-a]pyrimidine (III). Then the resultant compound is reacted with an appropriate halogenating agent, for instance, phosphorus oxychloride or phosphorus pentachloride, in a solvent, for instance, benzene, chloroform, carbon tetrachloride, or in the absence of solvent, at room temperature, or under heating or by refluxing under heating if necessary, to obtain 7-chloro-2-chloromethyl-2-triazolo[1,5-a]pyrimidine (IV). The resultant halogenated derivative of s-triazolo[1,5-a]pyrimidine thus obtained is dissolved in a solvent, for instance, ethanol or methanol, and if necessary a base such as triethylamine, dimethylaniline or pyridine as a deacidifying agent is added and reacted with addition of diethylamine. Selective reaction at the 7-position may be accomplished by controlling the amount of amine, temperature of reaction and reaction time. 2-Chloromethyl-7-diethylamino-5-methyl-s-triazolo[1,5-a]pyrimidine (V) thus obtained is dissolved in a solvent, for instance, methanol or ethanol, and if necessary a base, for instance triethylamine, dimethylaniline or pyridine as a deacidifying agent is added thereto. After the addition of N-n-propylaniline, reflux is carried out to obtain 7-diethylamino-5-methyl-2-(N-phenyl-N-n-propyl)aminomethyl-s-triazolo[1,5-a]pyrimidine (VI).

Another example is shown below:

3-Amino-5-hydroxymethyl-s-triazole is condensed with a 1,3-diketone compound, for instance, with α-acetyl-γ-butylolactone and cyclized in the presence of a base catalyst to obtain 7-hydroxy-6-(2-hydroxyethyl)-2-hydroxymethyl-5-methyl-s-triazolo[1,5-a]pyrimidine which is halogenated with an appropriate halogenating agent to obtain a 7-halo-6-(2-haloethyl)-2-halomethyl-5-methyl-s-triazolo[1,5-a]pyrimidine. This halogenated compound is then reacted with an appropriate alkylamine to obtain an 8-alkyl-2-halomethyl-5-methyl-6,7-dihydro-8H-pyrrolo[3,2-e]-s-triazolo[1,5-a]pyrimidine. Further, by treating the resultant compound with an appropriate amine, the desired pyrrolo[3,2-e]-s-triazolo[1,5-a]pyrimidine derivatives are obtained. An example is shown as follows:

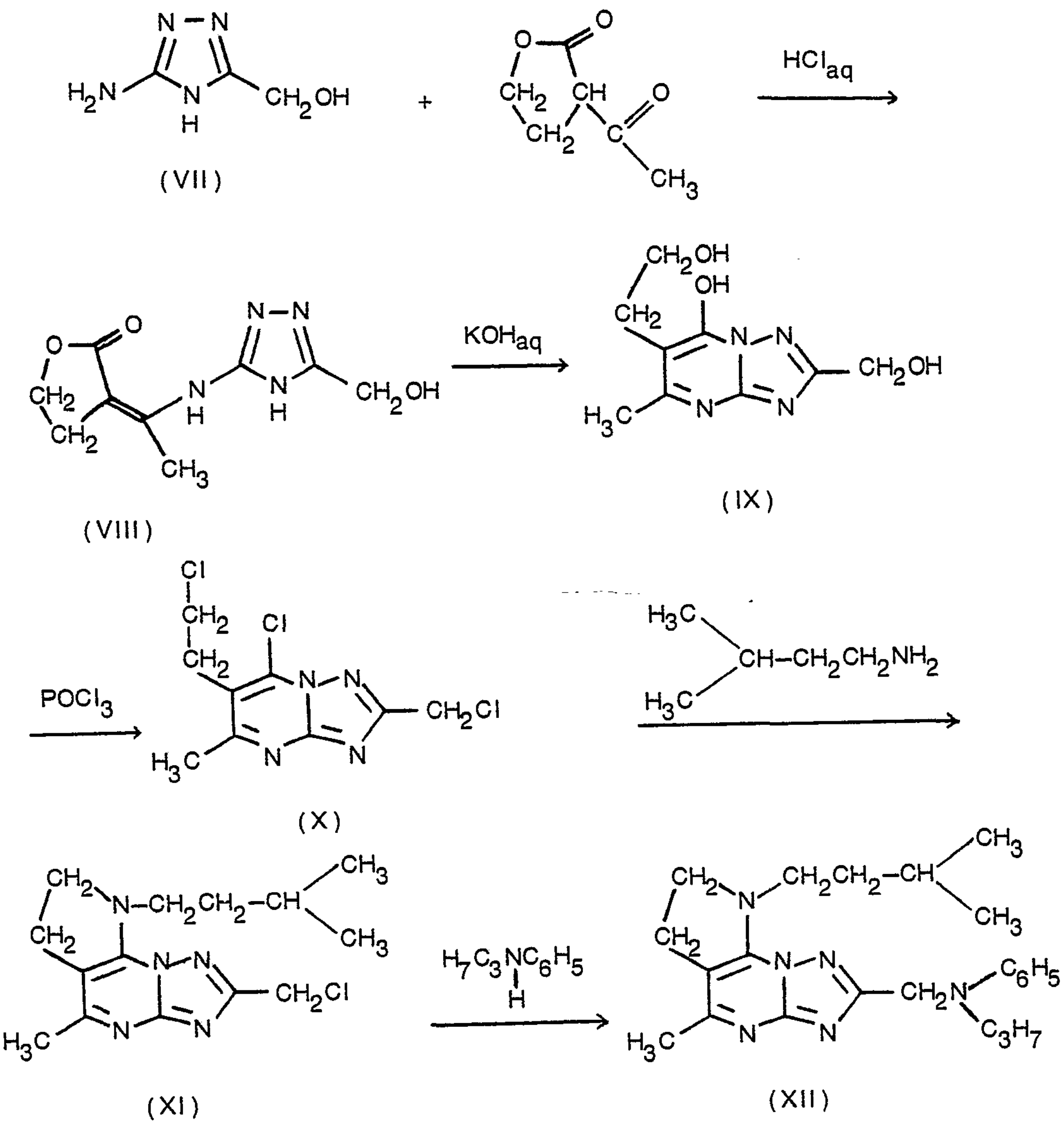


(VII) (VIII) (IX) (X) (XI) (XII)

3-Amino-5-hydroxymethyl-s-triazole (VII) was condensed with α-acetyl-γ-butylolactone in the presence of mineral acid, for instance, hydrochloric acid, at room temperature and cyclized using a metal hydroxide, for instance, potassium hydroxide to obtain 7-hydroxy-6-(2-hydroxyethyl)-2-hydroxymethyl-5-methyl-s-triazolo[1,5-a]pyrimidine (IX). The compound (IX) was refluxed under heating using an appropriate halogenating agent, for instance, phosphorus oxychloride or phosphorus pentachloride, in the absence of solvent or in an appropriate solvent, for instance, benzene, chloroform or carbon tetrachloride, to obtain 7-chloro-6-(2-chloroethyl)-2-chloromethyl-5-methyl-s-triazolo[1,5-a]pyrimidine (X). The compound (X) thus obtained was dissolved in a solvent, for instance, ethanol or methanol, and if necessary, a deacidifying agent, for instance, triethylamine, dimethylaniline or pyridine, was added, and refluxed with addition of isoamylamine. The selective formation of the pyrrolo ring may be attained by controlling the amount of amine, reaction temperature and reaction time. 2-Chloromethyl-8-isoamyl-5-methyl-6,7-dihydro-8H-pyrrolo[3,2-e]-s-triazolo[1,5-a]pyrimidine thus obtained was dissolved in a solvent, for instance, ethanol or methanol, and a deacidifying agent, for instance, triethylamine, dimethylaniline or pyridine, was added if necessary, and refluxed under heating with the addition of N-n-propylaniline to obtain 8-isoamyl-5-methyl-2-(N-phenyl-N-n-propyl)amino-6,7-dihydro-8H-pyrrolo[3,2-e]-s-triazolo[1,5-a]pyrimidine (XII).

The compounds, obtained by such procedures and represented by the formula (I), may be converted to their pharmacologically acceptable salts which are exemplified by acid adduct with inorganic acids, for instance, hydrochloric acid, hydrobromic acid, hydroiodic acid, sulphuric acid, nitric acid or phosphoric acid, or organic acids, for instance, maleic acid, citric acid, tartaric acid, oxalic acid or succinic acid.

All of the s-triazolo[1,5-a]pyrimidine derivatives of the present invention possess favourable characteristics as therapeutic agents, especially for cardiovascular diseases, since they have potent vasodilating action, hypotensive action, inhibitory action on platelet aggregation and lowering action on blood

cholesterol levels, and furthermore, are soluble in water. Thus, the compounds of the present invention, or their acid adducts may be formulated to products, which can be used therapeutically, alone or together with other pharmacologically active compounds, by any of the conventional methods using pharmaceutically acceptable binders, fillers or fragrances.

Examples of the process for preparing compounds of the present invention are shown below, but the present invention is not limited to them.

Example 1

7-diethylamino-5-methyl-2-(N-phenyl-N-n-propyl)aminomethyl-s-triazolo[1,5-a]pyrimidine

To 136 g of aminoguanidine bicarbonate was added 220 ml of glycolic acid 70% aqueous solution and 2 ml of concentrated nitric acid and the mixture was heated up to about 85°C. After the mixture was dissolved, the solution was refluxed under heating for 24 hours. After the completion of the reaction, the reaction mixture was cooled and precipitating crystals were filtered off. The crystals were recrystallized from ethanol to obtain 3-amino-5-hydroxymethyl-s-triazole (50 g, yield 51%, melting point 114—115°C).

40 g of 3-amino-5-hydroxymethyl-s-triazole and 43.5 g of ethyl acetoacetate were dissolved in 130 ml of acetic acid and refluxed under heating for 6 hours. After the completion of the reaction, the reaction mixture was cooled and precipitating crystals were filtered off. The crystals were recrystallized from ethanol to obtain 7-hydroxy-2-hydroxymethyl-5-methyl-s-triazolo[1,5-a]pyrimidine (76 g, yield 50%, melting point 276—278°C).

25 g of 7-hydroxy-2-hydroxymethyl-5-methyl-s-triazolo[1,5-a]pyrimidine was dissolved in 100 ml of phosphorus oxychloride and refluxed under heating for 3 hours. After the completion of the reaction, the reaction mixture was concentrated under reduced pressure and the residue was dissolved in 200 ml of chloroform. The chloroform solution was washed with 70 ml of water, then 70 ml of saturated solution of sodium bicarbonate. The solution was dried over sodium sulphate anhydride, and then the solvent was distilled off under reduced pressure. The residue was recrystallized from ethyl acetate to obtain 7-chloro-2-chloromethyl-5-methyl-s-triazolo[1,5-a]pyrimidine (25 g, yield 83%, melting point 264—266°C).

10 g of 7-chloro-2-chloromethyl-5-methyl-s-triazolo[1,5-a]pyrimidine, and 8 g of diethylamine were dissolved in 90 ml of ethanol and refluxed under heating for 3 hours. The reaction mixture was concentrated under reduced pressure, and the residue was dissolved in 50 ml of chloroform and washed with 10 ml of water. The chloroform solution was dried over sodium sulphate anhydride. The solvent was distilled off under reduced pressure, and the residue was recrystallized from mixture of methylene dichloride-normal hexane to obtain 2-chloromethyl-7-diethylamino-5-methyl-s-triazolo[1,5-a]pyrimidine (6 g, yield 45%, melting point 101—102°C).

NMR (δ: $CDCl_3$) 5.97 1H *s*, 4.71 2H *s*, 3.84 4H (*q*, 7Hz), 2.48 3H *s*, 1.33 6H (*t*, 7Hz)

2.4 g of 2-chloromethyl-7-diethylamino-5-methyl-s-triazolo[1,5-a]pyrimidine and 1.4 g of N-n-propylaniline and 1.5 g of triethylamine were dissolved in 50 ml of ethanol and refluxed under heating for 3 hours. The reaction mixture was concentrated under reduced pressure and the residue was dissolved into 100 ml of chloroform and washed with 100 ml of water. After drying the chloroform solution over sodium sulphate anhydride, the solvent was distilled off under reduced pressure, and the residue was purified by column chromatography on silica-gel to obtain 7-diethylamino-5-methyl-2-(N-phenyl-N-n-propyl)-s-triazolo[1,5-a]pyrimidine (1.8 g, yield 50%).

NMR (δ: $CDCl_3$) 7.41—6.74 5H *m*, 5.91 11H *s*, 4.74 2H *s*, 3.78 4H (*q*, 7Hz), 3.55 2H (*t*, 7Hz), 2.52 3H *s*, 1.77 2H (*m*, 7Hz), 1.28 6H (*t*, 7Hz), 1.02 3H (*t*, 7Hz)

Example 2

8-isoamyl-5-methyl-2-(N-phenyl-N-n-propyl)aminomethyl-6.7-dihydro-8H-pyrrolo[3.2-e]-s-triazolo[1,5-a]pyrimidine

270 g of 3-amino-5-hydroxymethyl-s-triazole and 456 g of α-acetyl-γ-butylolactone were dissolved in mixture of 60 ml of concentrated hydrochloric acid and 1.5 liter of water, and allowed for reaction at room temperature for two days. After the completion of the reaction, the reaction mixture was concentrated under reduced pressure, and then 2 liter of ethanol was added to it. The precipitated crystals were filtered off to obtain 3-[2-(3-hydroxymethyl-s-triazolo-2-yl)aminoethylidene]-γ-butylolactone (305 g, yield 87%, melting point 211—214°C).

400 g of 3-[2-(3-hydroxymethyl-s-triazolo-2-yl)aminoethylidene]-γ-butylolactone, and 158 g of potassium hydroxide were dissolved in 500 ml of water and allowed for reaction at room temperature for 2 days. After the reaction was completed, the reaction mixture was adjusted to pH 5—6 with acetic acid, and concentrated under reduced pressure to 1/3—1/5 of initial volume. The concentrate was cooled with ice for crystallization. The crystals were filtered off to obtain 7-hydroxy-2-hydroxymethyl-6-(2-hydroxyethyl)-5-methyl-s-triazolo[1,5-a]pyrimidine (240 g, yield 54%, melting point 275—277°C).

220 g of 7-hydroxy-2-hydroxymethyl-6-(2-hydroxyethyl)-5-methyl-s-triazolo[1,5-a]pyrimidine were dissolved in 1 liter of phosphorus oxychloride and refluxed under heating for 3 hours. Then the reaction mixture was concentrated under reduced pressure and the residue was dissolved in 3 liter of chloroform. The chloroform solution was washed, firstly with 100 ml of water then with 250 ml of saturated solution of sodium bicarbonate, and dried over sodium sulphate anhydride. The solvent was distilled off under reduced pressure, and the residue was recrystallized from ethyl acetate to obtain 7-chloro-6-(2-chloro-

ethyl)-2-chloromethyl-5-methyl-s-trizolo[1,5-a]pyrimidine (190 g, yield 80%, melting point 118—123°C).

124 g of 7-chloro-6-(2-chloroethyl)-2-chloromethyl-5-methyl-s-triazolo[1,5-a]pyrimidine, 39.0 g of isoamylamine and 43.7 g of triethylamine were dissolved in 1.5 liter of ethanol and refluxed under heating for 3 hours. Then the reaction mixture was concentrated under reduced pressure, and the residue was dissolved in 1.5 liter of chloroform. The chloroform solution was washed with 200 ml of water and dried over sodium sulphate anhydride, then the solvent was distilled off under reduced pressure. The residue was recrystallized from mixture of methylene dichlorode-normal hexane to obtain 2-chloromethyl-8-isoamyl-5-methyl-6,7-dihydro-8H-pyrrolo[3,2-e]-s-triazolo[1,5-a]pyrimidine (81 g, yield 80%, melting point 113—115°C).

NMR (δ: $CDCl_3$) 4.52 2H *s*, 3.91 2H (*t*, 7Hz), 3.70 2H (*t*, 7Hz), 2.81 2H (*t*, 9Hz), 2.11 3H *s*, 1.70—1.40 3H *m*, 0.96 6H (*d*, 7Hz)

25 g of 2-chloromethyl-8-isoamyl-5-methyl-6,7-dihydro-8H-pyrrolo[3,2-e]-s-triazolo[1,5-a]pyrimidine, 15 g of N-normal propylaniline and 11 g of triethylamine were dissolved in 200 ml of ethanol and refluxed under heating for 10 hours. Then the reaction mixture was concentrated under reduced pressure, and the residue was dissolved in 500 ml of chloroform. This chloroform solution was washed with 80 ml of water and dried over sodium sulphate anhydride, then the solvent was distilled off under reduced pressure. The residue was purified by column chromatography on silica-gel to obtain 8-isoamyl-5-methyl-2-(N-phenyl-N-n-propyl)aminomethyl-6,7-dihydro-8H-pyrrolo[3,2-e]-s-triazolo[1,5-a]pyrimidine (12 g, yield 40%, melting point 117—121°C).

NMR (δ: $CDCl_3$) 7.1—6.6 5H *m*, 4.64 2H *s*, 4.00 2H (*t*, 7Hz), 3.77 2H (*t*, 9Hz), 3.51 2H (*t*, 7Hz), 3.00 2H (*t*, 9Hz), 2.31 3H *s*, 1.8—1.3 5H *m*, 0.90 6H (*d*, 7Hz)

## Example 3

8-isopropyl-5-methyl-2-(N-phenyl-N-n-propyl)aminomethyl-6,7-dihydro-8H-pyrrolo[3,2-e]-s-triazolo[1,5-a]pyrimidine

41 g of 7-chloro-6-(2-chloroethyl)-5-methyl-s-triazolo[1,5-a]pyrimidine, 9 g of isopropylamine and 15 g of triethylamine were dissolved in 500 ml of ethanol and reacted in closed container at 80°C for 10 hours. After the reaction was completed, the solvent was distilled off under reduced pressure, and the residue was dissolved in 300 ml of chloroform. The chloroform solution was washed with 80 ml of water and dried over sodium sulphate anhydride, then the solvent was distilled off under reduced pressure. The residue was recrystallized from 500 ml of n-hexane to obtain 2-chloromethyl-8-isopropyl-5-methyl-6,7-dihydro-8H-pyrrolo[3,2-e]-s-triazolo[1,5-a]pyrimidine (31 g, yield 80%, melting point 172—173°C).

NMR (δ: $CDCl_3$) 5.58 1H (*m* 7Hz), 4.69 2H *s*, 3.84 2H (*t*, 9Hz), 3.08 2H (*t*, 9Hz), 2.35 3H *s*, 1.30 6H (*d*, 7Hz)

20 g of 2-chloromethyl-8-isopropyl-5-methyl-6,7-dihydro-8H-pyrrolo[3,2-e]-s-triazolo[1,5-a]pyrimidine and 7 g of N-n-propylaniline and 5 g of triethylamine were dissolved in 300 ml of ethanol and refluxed for 12 hours. Then the solvent was distilled off under reduced pressure, and the residue was dissolved in 100 ml of chloroform. This chloroform solution was washed with 30 ml of water and dried over sodium sulphate anhydride, then the solvent was distilled off under reduced pressure. The residue was purified by column chromatography on silica-gel to obtain 8-isopropyl-5-methyl-2-(N-phenyl-N-n-propyl)aminomethyl-6,7-dihydro-8H-pyrrolo[3,2-e]-s-triazolo[1,5-a]pyrimidine (8.2 g, yield 38%, melting point 90—92°C).

NMR (δ: $CDCl_3$) 7.31—6.81 5H *m*, 5.49 1H (*m*, 7Hz), 4.64 2H *s*, 3.78 2H (*t*, 9Hz), 3.50 2H (*t*, 7Hz), 3.00 2H (*t*, 9Hz), 2.32 3H *s*, 1.22 6H (*d*, 7Hz), 0.95 3H (*t*, 7Hz)

## Example 4

8-n-butyl-5-methyl-2-(N-phenyl-N-n-propyl)aminomethyl-6,7-dihydro-8H-pyrrolo[3,2-e]-s-triazolo[1,5-a]pyrimidine

21 g of 7-chloro-6-(2-chloroethyl)-2-chloromethyl-5-methyl-6,7-dihydro-8H-pyrrolo[3,2-e]-s-triazolo[1,5-a]pyrimidine, 5.46 g of n-butylamine and 8.2 g of triethylamine were dissolved in 400 ml of ethanol and refluxed under heating for 3 hours. Then the solvent was distilled off under reduced pressure, and the residue was dissolved in 300 ml of chloroform. This chloroform solution was washed with 70 ml of water and dried over sodium sulphate anhydride, then the solvent was distilled off under reduced pressure. The residue was recrystallized from 500 ml of n-hexane to obtain crystals of 2-chloromethyl-8-n-butyl-5-methyl-6,7-dihydro-8H-pyrrolo[3,2-e]-s-triazolo[1,5-a]pyrimidine (18 g, yield 85%, melting point 104—105°C).

NMR (δ: $CDCl_3$) 4.71 2H *s*, 4.06 2H (*t*, 7Hz), 3.89 2H (*t*, 9Hz), 3.09 2H (*t*, 9Hz), 2.33 3H *s*, 1.75—1.25 4H *m*, 0.97 3H (*t*, 7Hz)

8 g of 2-chloromethyl-8-n-butyl-5-methyl-6,7-dihydro-8H-pyrrolo[3,2-e]-s-triazolo[1,5-a]pyrimidine, 4 g of N-n-propylaniline and 3.2 g of triethylamine were dissolved in 300 ml of ethanol and refluxed under heating for 10 hours. Then the solvent was distilled off under reduced pressure, and the residue was dissolved in 200 ml of chloroform. This chloroform solution was washed with 60 ml of water and dried over sodium sulphate anhydride, then the solvent was distilled off under reduced pressure. The residue was purified by column chromatography on silica-gel to obtain 8-n-butyl-5-methyl-2-(N-phenyl-N-n-propyl)aminomethyl-6,7-dihydro-8H-pyrrolo[3,2-e]-s-triazolo[1,5-a]pyrimidine (4.8 g, yield 40%, melting point 88—92°C).

NMR (δ: $CDCl_3$) 7.10—6.63 5H *m*, 4.63 2H *s*, 3.85 2H (*t*, 7Hz), 3.52 2H (*t*, 9Hz), 3.10 2H (*t*, 9Hz), 2.35 3H *s*, 1.70—1.22 6H *m*, 0.95 3H (*t*, 7Hz).

Example 5

8-isoamyl-5-methyl-2-(N-methyl-N-phenyl)aminomethyl-6,7-dihydro-8H-pyrrolo[3,2-e]-s-triazolo[1,5-a]pyrimidine

20 g of 2-chloromethyl-8-isoamyl-5-methyl-6,7-dihydro-8H-pyrrolo[3,2-e]-s-triazolo[1,5-a]pyrimidine, 7 g of N-methylaniline and 7 g of triethylamine were dissolved in 200 ml of ethanol and refluxed under heating for 10 hours. Then the solvent was distilled off under reduced pressure, and the residue was dissolved in 200 ml of chloroform. This chloroform solution was washed with 50 ml of water and dried over sodium sulphate anhydride, then the solvent was removed under reduced pressure. The residue was purified by column chromatography on silica-gel to obtain 8-isoamyl-5-methyl-2-(N-methyl-N-phenyl)aminomethyl-6,7-dihydro-8H-pyrrolo[3,2-e]-s-triazolo[1,5-a]pyrimidine (8.4 g, yield 36%, melting point 91—101°C).

NMR (δ: $CDCl_3$) 7.26—6.72 5H *m*, 4.67 2H *s*, 4.09 2H (*t*, 7Hz), 3.80 2H (*t*, 9Hz), 3.19 3H *s*, 2.95 2H (*t*, 9Hz), 2.36 3H *s*, 1.70—1.40 3H *m*, 0.98 6H (*d*, 7Hz)

Example 6

8-isoamyl-2-(N-isoamyl-N-phenyl)aminomethyl-5-methyl-6,7-dihydro-8H-pyrrolo[3,2-e]-s-triazolo[1,5-a]pyrimidine

20 g of 2-chloromethyl-8-isoamyl-5-methyl-6,7-dihydro-8H-pyrrolo[3,2-e]-s-triazolo[1,5-a]pyrimidine, 11 g of isoamylaniline and 9 g of triethylamine were dissolved in 300 ml of ethanol and refluxed under heating for 10 hours. Then the solvent was distilled off under reduced pressure, and the residue was dissolved in 200 ml of chloroform. This chloroform solution was washed with 50 ml of water and dried over sodium sulphate anhydride, then the solvent was distilled off. The residue was purified by column chromatography on silica-gel to obtain 8-isoamyl-2-(N-isoamyl-N-phenyl)aminomethyl-6,7-dihydro-8H-pyrrolo[3,2-e]-s-triazolo[1,5-a]pyrimidine (7.3 g, yield 30%, melting point 109—110°C).

NMR (δ: $CDCl_3$) 7.26—6.50 5H *m*, 4.63 2H *s*, 3.99 2H (*t*, 7Hz), 3.79 2H (*t*, 9Hz), 3.52 2H (*t*, 7Hz), 3.06 2H (*t*, 9Hz), 2.33 3H *s*, 1.75—1.30 6H *m*, 0.96 6H (*d*, 7Hz), 0.90 6H (*d*, 7Hz)

**Claims**

1. A s-triazolo[1,5-a]pyrimidine derivative which is represented by the general formula (I):

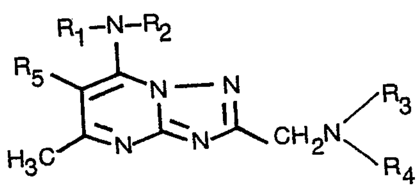

(I)

wherein

$R_1$ and $R_2$, which are identical or different, each represents a straight- or branched-chained alkyl radical having from one to six carbon atoms or a hydrogen atom;

$R_3$ represents straight- or branched-chained alkyl radical having from one to six carbon atoms;

$R_4$ represents a phenyl radical; and

$R_5$ represents a hydrogen atom or may form a $—CH_2—CH_2—$ bond together with $R_1$.

2. A compound according to claim 1, wherein $R_1$ and $R_2$ are ethyl and $R_3$ is n-propyl.

3. A compound according to claim 1, wherein $R_1$ and $R_5$ together form $—CH_2—CH_2—$, $R_2$ is isoamyl and $R_3$ is n-propyl.

4. A compound according to claim 1, wherein $R_1$ and $R_5$ together form $—CH_2—CH_2—$, $R_2$ is isopropyl and $R_3$ is n-propyl.

5. A compound according to claim 1, wherein $R_1$ and $R_5$ together form $—CH_2—CH_2—$, $R_2$ is n-butyl and $R_3$ is n-propyl.

6. A compound according to claim 1, wherein $R_1$ and $R_5$ together form $—CH_2—CH_2—$, $R_2$ is isoamyl and $R_3$ is methyl.

7. A compound according to claim 1, wherein $R_1$ and $R_5$ together form $—CH_2—CH_2—$, $R_2$ is isoamyl and $R_3$ is isoamyl.

**Patentansprüche**

1. s-Triazolo-[1,5-a]-pyrimidin-Derivat der allgemeinen Formel (I)

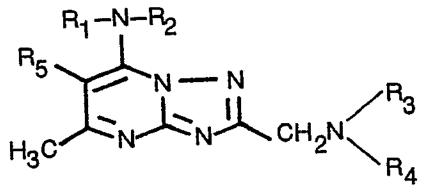

(I)

in der

$R_1$ und $R_2$, die gleich oder verschieden sind, jeweils einen geradkettigen oder kettenverzweigten Alkyl-Rest mit einem bis sechs Kohlenstoff-Atomen oder ein Wasserstoff-Atom bezeichnen,

$R_3$ einen geradkettigen oder kettenverzweigten Alkyl-Rest mit einem bis sechs Kohlenstoff-Atomen bezeichnet,

$R_4$ einen Phenyl-Rest bezeichnet und

$R_5$ ein Wasserstoff-Atom bezeichnet oder zusammen mit $R_1$ eine —$CH_2$—$CH_2$— Bindung bilden kann.

2. Verbindung nach Anspruch 1, dadurch gekennzeichnet, daß $R_1$ und $R_2$ Ethyl sind und $R_3$ n-Propyl ist.

3. Verbindung nach Anspruch 1, dadurch gekennzeichnet, daß $R_1$ und $R_5$ zusammen —$CH_2$—$CH_2$— bilden, $R_2$ Isoamyl ist und $R_3$ n-Propyl ist.

4. Verbindung nach Anspruch 1, dadurch gekennzeichnet, daß $R_1$ und $R_5$ zusammen —$CH_2$—$CH_2$— bilden, $R_2$ Isopropyl ist und $R_3$ n-Propyl ist.

5. Verbindung nach Anspruch 1, dadurch gekennzeichnet, daß $R_1$ und $R_5$ zusammen —$CH_2$—$CH_2$— bilden, $R_2$ n-Butyl ist und $R_3$ n-Propyl ist.

6. Verbindung nach Anspruch 1, dadurch gekennzeichnet, daß $R_1$ und $R_5$ zusammen —$CH_2$—$CH_2$— bilden, $R_2$ Isoamyl ist und $R_3$ Methyl ist.

7. Verbindung nach Anspruch 1, dadurch gekennzeichnet, daß $R_1$ und $R_5$ zusammen —$CH_2$—$CH_2$— bilden, $R_2$ Isoamyl ist und $R_3$ Isoamyl ist.

**Revendications**

1. Dérivés de s-triazolo 1,5-a pyrimidine représentée par la formule générale (I):

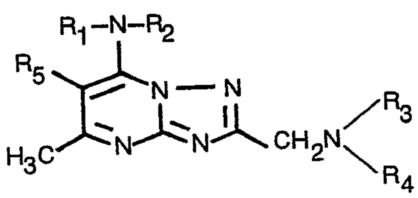

(I)

dans laquelle

$R_1$ et $R_2$ qui sont identiques ou différents, représentent chacun un groupe alkyle à chaîne droite ou ramifiée ayant de 1 à 6 atomes de carbone, ou un atome d'hydrogène;

$R_3$ représente un groupe alkyle à chaîne droite ou ramifiée ayant de 1 à 6 atomes de carbone;

$R_4$ représente un groupe phényle; et

$R_5$ représente un atome d'hydrogène ou peut former une liaison —$CH_2$—$CH_2$— avec $R_1$.

2. Composé selon la revendication 1, dans lequel $R_1$ et $R_2$ sont un groupe éthyle et $R_3$ est un groupe n-propyle.

3. Composé selon la revendication 1, dans lequel $R_1$ et $R_5$ forment ensemble le groupe —$CH_2$—$CH_2$—, $R_2$ est un groupe isoamyle et $R_3$ est un groupe n-propyle.

4. Composé selon la revendication 1, dans lequel $R_1$ et $R_5$ forment ensemble le groupe —$CH_2$—$CH_2$—, $R_2$ est un groupe isopropyle et $R_3$ est un groupe n-propyle.

5. Composé selon la revendication 1, dans lequel $R_1$ et $R_5$ forment ensemble le groupe —$CH_2$—$CH_2$, $R_2$ est un groupe n-butyle et $R_3$ est un groupe n-propyle.

6. Composé selon la revendication 1, dans lequel $R_1$ et $R_5$ forment ensemble le groupe —$CH_2$—$CH_2$—, $R_2$ est un groupe isoamyle et $R_3$ est un groupe méthyle.

7. Composé selon la revendication 1, dans lequel $R_1$ et $R_5$ forment ensemble le groupe —$CH_2$—$CH_2$, $R_2$ est un groupe isoamyle et $R_3$ est un groupe isoamyle.